# EUROPEAN PATENT SPECIFICATION

(11) **EP 0 435 767 B1**
(45) Date of publication and mention of the grant of the patent: **04.05.1994**
(21) Application number: 90403763.7
(22) Date of filing: 26.12.1990
(51) Int. Cl.: C12M 1/34, C12Q 1/06

(54) **Method for determination of aerobic bacteria and apparatus therefor**
Verfahren und Vorrichtung zur Bestimmung von aeroben Bakterien
Méthode et appareil pour la détermination de bactéries aérobiques

(30) Priority: 27.12.1989 JP 340063/89
(43) Date of publication of application: 03.07.1991
(73) Proprietor: SHIMADZU CORPORATION, Nakagyo-ku Kyoto-shi Kyoto 604 (JP)
(72) Inventor: Kawabata, Nariyoshi, Osaka 557 (JP)
(74) Representative: Orès, Bernard

(56) References cited:
- EP-A- 360 276
- JP-A-62 041 641
- US-A- 4 517 291
- US-A- 4 614 716
- PATENT ABSTRACTS OF JAPAN, vol. 6, no. 19 (C-90)(897) 03 February 1982#
- PATENT ABSTRACTS OF JAPAN; vol. 13; no. 419 (C-637)(3767) 18 September 1989#

## Description

This Invention relates to a method for the detection of aerobic bacteria and an apparatus therefor, and more particularly to a method for a fast-response determination of the viable cell number (activity) of aerobic bacteria and an apparatus therefor.

The most commonly used method for determining concentration of viable bacteria present in various samples is to visually observe colonies prepared by aseptically carrying out a stationary culture of a constant amount of samples on an agar media at least more than one day to calculate the viable cell number on the basis of the number of colonies. Alternatively, the viable cell number can be determined by a method in that microbial cells are first extracted from a sample which are added to a dye solution for staining, and thereafter the stained cell number is counted through a microscope.

However, in the former method, it is quite cumbersome and time consuming to dilute a sample to a fixed dilution ratio in an aseptic condition, carry out a stationary culture for several days and visually count the generated colonies. In the latter method, dead bacteria contained in samples, various solid matters in the culture medium and the like are stained in a similar way as viable bacteria, so that it is quite hard to accurately determine the viable cell number of bacteria.

A method for determining the viable cell number of bacteria and the like on the basis of enzymatic measurement of catalase activity of bacteria or yeast has been proposed (Japanese Examined Published Patent Application No 15999/1980, and US Patent No 3,383,034. But, this conventional process includes the disadvantages that it requires the extraction of bacteria and the like from sample, it is cumbersome to set conditions conformable for enzyme reaction, and it is hard to achieve a fast-response determination.

JP 56 140 898 describes an apparatus comprising 2 compartments : in one compartment, an electrode is placed in a phosphoric acid buffer solution comprising a nutrient source, and saturated with oxygen ; in the other, the bacteria are kept alive in a specimen solution. The compartments are separated by a membrane allowing the passage of the nutrients and oxygen, but not of bacteria. The number of living cells is determined from the level of the current measured by the oxygen electrode.

Indeed, it has been strongly desired to quickly measure the concentration of the viable cell in production control of fermentation and quality control of perishable foodstuff. However, as aforementioned, there is not yet known a practical determination method which meets the above needs.

An object of the Invention is to provide a method and apparatus for promptly and accurately determining the viable cell number of aerobic bacteria present in a sample using a simple operation.

JP 62 041 641 describes the use of vinyl polymers having a substituted pyridinium group as a microbial adsorbent for control of microorganism.

EP 0 360 276 which is to be considered as comprised in the state of the art according to Article 54(3) and (4) CBE, describes a microbial sensor consisting of a container packed with a microbial adsorbent comprising a polymer containing a pyridinium group of formula (A) : 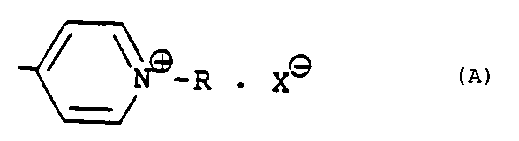
wherein R represents a benzyl group, a phenethyl group, an alkyl group having from 1 to 16 carbon atoms, or a pentafluorophenyl methyl group, and X represents a halogen atom, and an electrochemical detection device, such as an oxygen electrode. The container and the detection device are provided separately in the microbial sensor.

The present invention has been achieved by the inventor's discovery of the fact that an appropriate water-insoluble pyridinium resin is capable of intensively capturing bacteria in their live state.

The water-insoluble pyridinium type resin used in the present invention is a vinyl copolymer composed of the component units represented by the following formulas (I) and (II), or a cross-linked vinyl polymer formed by cross-linking the polymer composed of the component unit of the formula (I) by use of a polymeric chain composed of the unit of the formula (II) 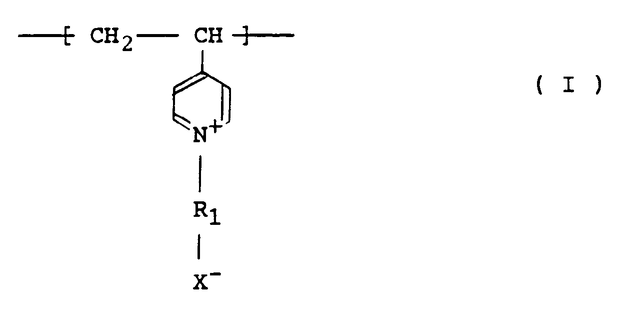 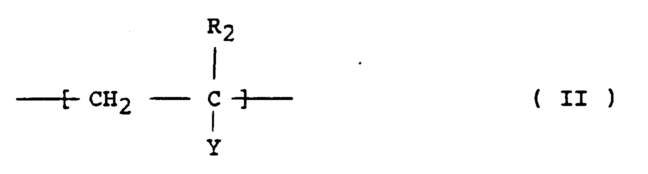
wherein R₁ represents a benzyl group, an alkyl group having C₄ to C₁₆, or an aryl group, R₂ represents an alkyl group having C₁ to C₃, X represents a halogen atom, and Y represent a hydrogen atom, an alkyl group having C₁ to C₃, a benzyl group, an aryl group, an ether group, a carboxyl group, or a carboxylic ester group, and the molar ratio of the component unit (I) to (II) is of from 1 : 20 to 1 : 0.01.

This ratio allows to effectively capture bacteria while keeping the water insolubility.

A polymerization degree of more than 100 should usually be considered as sufficient, excepting the cross-linked polymer wherein it is difficult to define the degree.

The above water-insoluble pyridinium type resin may be prepared from the corresponding monomers in accordance with a customary polymerization technique or cross-linking process. Alternatively, a commercially available water-insoluble pyridinium type resin in the form of powder, particle, membrane, fiber and the like may be employed.

According to the present invention, there is provided a method for determining viable cell number of aerobic bacteria which comprises placing a layer of the above water-insoluble pyridinium type resin on the sensing portion of an oxygen-sensing electrode, subjecting an aqueous bacteria-containing sample to be in contact with the water-insoluble pyridinium type resin layer, and determining the viable cell number of aerobic bacteria in the sample on the basis of an output of the oxygen-sensing electrode detected upon the contact of the sample with the pyridinium type resin layer.

Further, according to the present invention, there is provided an apparatus for determining aerobic bacteria which comprises an oxygen-sensing electrode having an oxygen-sensing portion and a flow cell capable of passing an aqueous sample therethrough while the sample is in contact with the oxygen-sensing portion, the flow cell being filled with the water-insoluble pyridinium type resin mentioned above.

The oxygen-sensing electrode to be used in the present invention may suitably be a diaphragm type oxygen-sensing electrode.

The determination of the present invention may be carried out in such a manner that the water insoluble pyridinium type resin is first filled in a cell for measurement and a sample is fed in the cell with the sensing portion of the oxygen-sensing electrode being inserted therein. It is generally preferable to use an apparatus comprising an oxygen-sensing electrode with an oxygen-sensing portion provided with a flow cell filled with the water insoluble pyridinium type resin, so that the determination method of the present invention can be performed by passing the sample through the flow cell in a predetermined time. The time for passing the sample is suitably 10 to 30 min, and an aerated sample may be introduced into the flow cell.

According to the present invention, the viable cell number (activity) of various aerobic bacterial present in the sample can be quickly determined on the basis of an output of the above oxygen-sensing electrode. The aerobic bacteria to be determined by the present invention means generic aerobic bacteria which need and consume oxygen in their growth, including a so-called facultatively anaerobic bacteria but exclude anaerobic bacteria which do not at all consume oxygen.

In the method of the present invention, an aqueous sample containing bacteria is directed to contact with the water-insoluble pyridinium type resin to cause the resin to quantitatively capture on its surface bacterial cells in their live state. The captured living aerobic bacteria consume oxygen. The consumption of oxygen is correspondingly detected as a reduction of an electric current value (output) by the oxygen-sensing electrode. The consumption of oxygen is proportional to the number of captured and live bacteria, so that reduction of the electric current value is to be proportional to concentration of live bacteria. Although the insoluble pyridinium type resin also captures dead bacterial cells, the captured dead bacteria do not consume oxygen and cause no reduction of the electric current value. Hence, the viable cell number (activity) present in the sample can directly be determined on the basis of the reduction of electric current value (output), thereby enabling a fast-response and accurate determination of aerobic bacteria with a simple operation.

Figs. 1(a) and 1(b) show an example of an apparatus for determination of aerobic bacteria of the present invention ; in which Fig. 1(a) is an explosive explanatory view showing the apparatus and Fig. 1(b) is an explanatory view showing the construction of the apparatus assembled.

Fig. 2 is an explanatory view illustrating a determination system for performing the method for determination of aerobic bacteria in accordance with the present invention.

Fig. 3 is a graph clarifying a responsibility by the method of the present invention, and

Figs. 4 through 6 are graphs, each showing a relationship between the viable cell number (concentration) and the current reduction velocities of the oxygen-sensing electrode.

Next, specific examples of the determination method of the present invention by the use of the aerobic bacteria detection apparatus 1 of the present invention as shown in Figs.l(b) will be detailed.

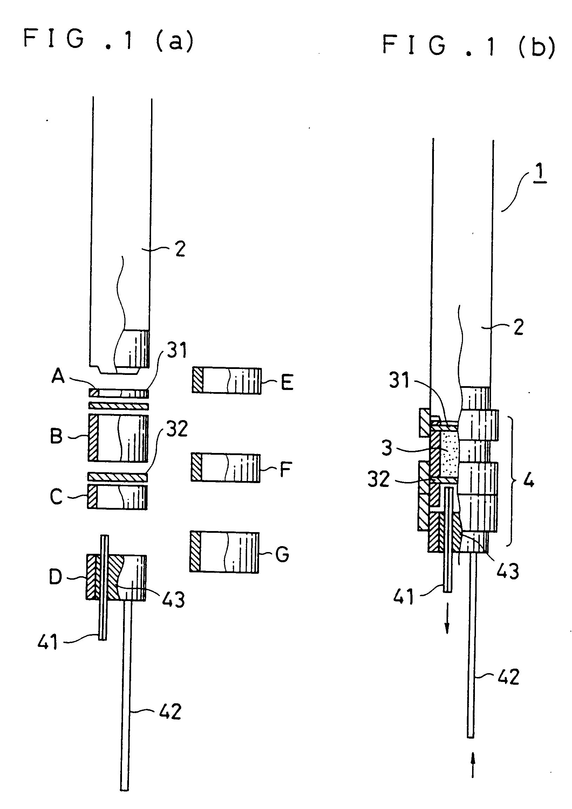

First, as shown in Figs. 1(a) and 1(b), a flow cell 4 is mounted to a sensing portion of a diaphragm type oxygen-sensing electrode 2 constructed by acrylic resin pipes B, C and D (8mm of inner diameter and 12mm of outer diameter), stainless pipes 41, 42 (1mm of inner diameter and 2mm of outer diameter), nylon meshes 31, 32 (100 meshes Japanese Industrial Standard), a double-sided adhesive A, and sealing silicon tubes E, F and G (10mm of inner diameter and 14mm of outer diameter). Upon assembly of the flow cell 4, beads of a cross-linked polymer of poly-N-benzyl-4-vinyl-pyridinium bromide (cross-linked BVP resin) was filled between the nylon meshes 31 and 32 in the flow cell 4 to form a water-insoluble pyridinium type resin filled layer 3, thereby constituting the aerobic bacteria determination apparatus 1 of the present invention. In the drawings, the reference numeral 43 designates an epoxy resin adhesive (curing in 12 hours) for sealing and fixing the stainless pipes 41 and 42.

The cross-linked BVP resin used in this embodiment comprises water insoluble beads of 0.2 mm in particle size which have been subjected to swelling by 0.05 M of phosphate buffer at pH 7.0 for more than one week before being filled in the device. Also, the resin filled layer 3 is 8 mm in diameter and 7.5 mm in length.

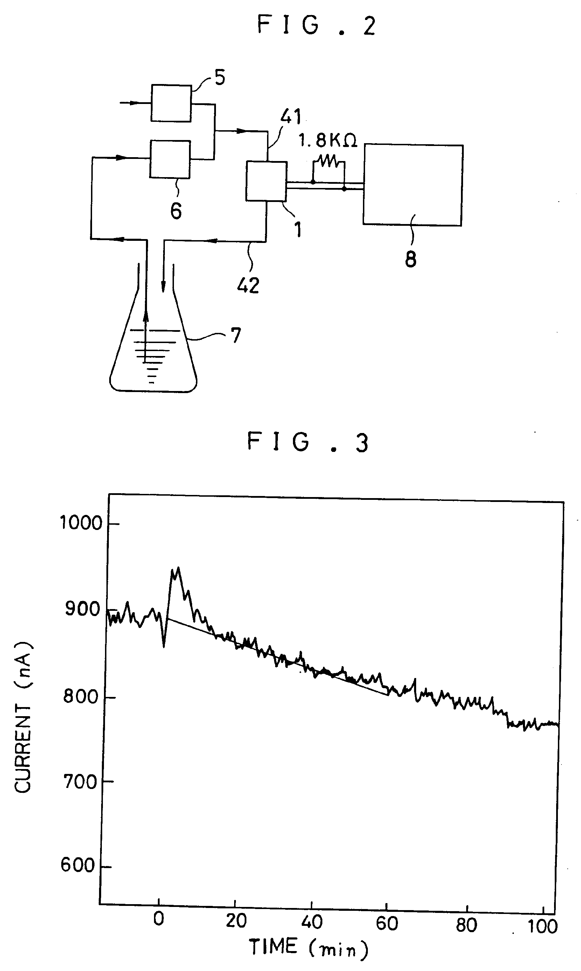

A detection system (kept at 25°C) was constituted by using the above determination apparatus 1 as shown in Fig. 2, wherein reference numeral 5 designates an air pump for aeration, 6 a peristaltic pump, 7 a reference liquid or a sample liquid reservoir, and 8 a recorder.

Air and 0.05 M of phosphate buffer of pH 7.0 (a reference liquid) were passed through the determination system (flow line) at 4 ml/min. When the current value of the recorder became constant, the phosphate buffer was switched to a sample liquid, namely, a suspension of bacteria for determination. The phosphate buffer and the sample of bacteria suspension used were each allowed to circulate through the system. Then, the current value reduced as time elapsed. The reduction velocity of the current value detected by the electrode was evaluated by nA/h. Since the capture of bacterial cells by the insoluble pyridinium type resin is quite intensive and irreversible, it is preferable to replace the resin used for each measurement.

### Example 1

A profile of reduction of an electric current value at the oxygen-sensing electrode upon feeding of E. coli (Escherichia coli) suspension having an optical density of 0.2 at 610nm into the above system is shown in Fig. 3, which reveals a current reduction velocity of 90 nA/h.

### Example 2

When E. coli (Escherichia coli) suspension having an optical density of 0.1 at 610nm was fed into the above system, a current reduction velocity was detected to be 58 nA/h.

### Example 3

When E. coli (Escherichia coli) suspension having an optical density of 0.1 at 610nm was sterilized at 121°C by use of an autoclave for 20 min and then fed into the above system, no substantial current reduction over the analytically allowable error range was observed.

### Example 4

When E. coli (Escherichia coli) suspension having an optical density of 0.7 at 610 nm was fed into the above system, a current reduction velocity was detected to be 428 nA/h.

### Example 5

When Pseudomonas aeruginosa suspension having an optical density of 0.1 at 610 nm was fed into the above system, a current reduction velocity was detected to be 39 nA/h.

### Example 6

When Staphylococcus aureus suspension having an optical density of 0.1 at 610 nm was fed into the above system, a current reduction velocity was detected to be 38 nA/h.

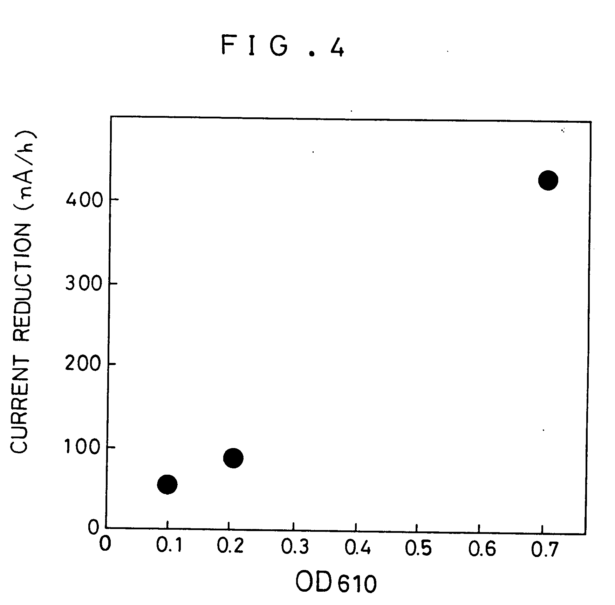

A calibration curve obtained from the result of the Examples 1, 3 and 4 is shown in Fig. 4.

### Example 7

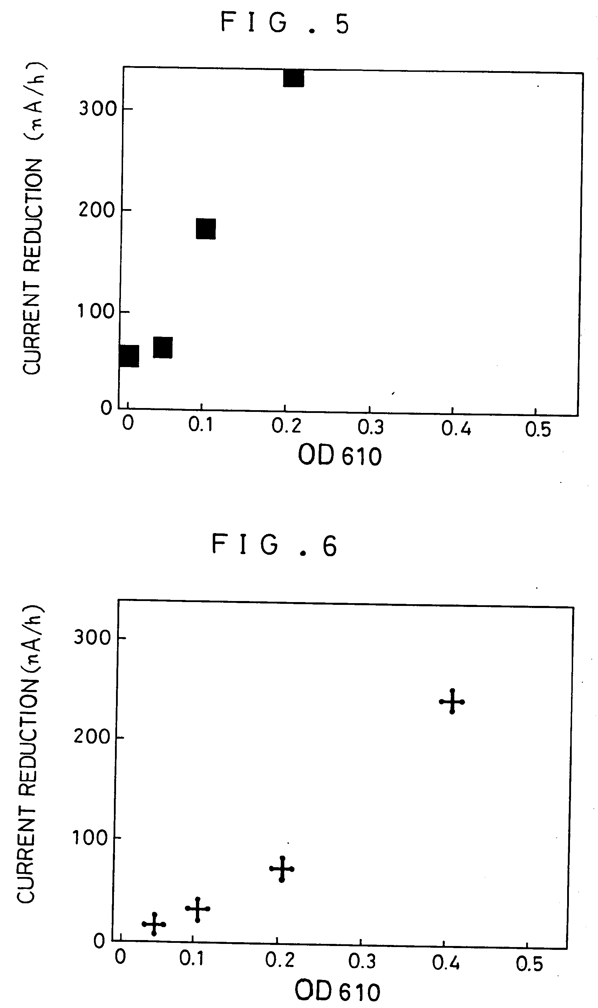

Bacillus subtilis suspension of 0.01, 0.05, 0.1 and 0.2 in optical density at 610 nm were each fed into the above system, and specific current reduction velocities were observed. A relationship between the observed current reduction velocities and corresponding absorption (O.D) is as shown in Fig. 5.

### Example 8

Saccharomyces cereisiae suspension of 0.05, 0.1, 0.2 and 0.4 in optical density at 610 nm were fed into the above system, and specific current reduction velocities were observed. A relationship between the observed current reduction velocities and corresponding absorption is as shown in Fig. 6.

These examples show an excellent linearity of the velocity to OD as seen, so that the determination of the viable cell number can be accurately and promptly effected.

As explained as above, the aerobic bacteria determination method and apparatus of the present invention realizes a fast-response and simply-operated determination of the viable cell number (activity) of aerobic bacteria present in various aqueous sample.

Therefore, the present invention has a quite large utility particularly in process control of fermentation and quality control of foodstuff.

## Claims

1. A method for determining viable cell number of aerobic bacteria, comprising :
- placing a water-insoluble pyridinium type resin layer on a sensing portion of an oxygen-sensing electrode ;
- contacting an aqueous bacteria-containing sample with the water-insoluble pyridinium type resin layer comprising a vinyl copolymer composed of the component units represented by the following formulas (I) and (II), or a cross-linked vinyl polymer formed by cross-linking a polymer composed of the component unit of the formula (I) with a polymeric chain composed of the unit of the formula (II) 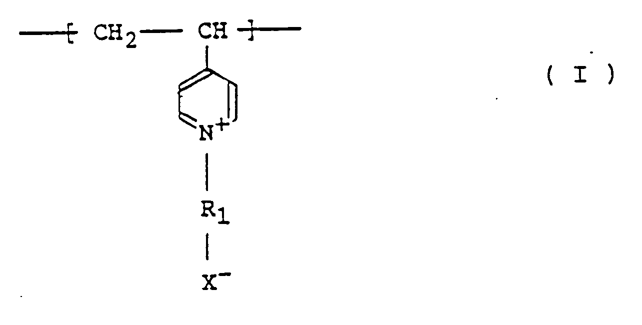 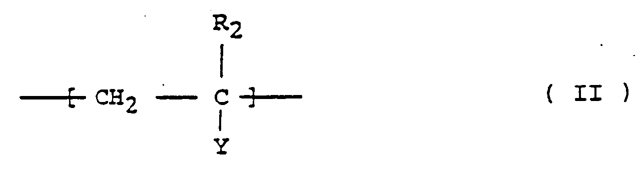 wherein R₁ represents a benzyl group, an alkyl group having C₄ to C₁₆, or an aryl group, R₂ represents an alkyl group having C₁ to C₃, X represents a halogen atom, and Y represent a hydrogen atom, an alkyl group having C₁ to C₃, a benzyl group, an aryl group, an ether group, a carboxyl group, or a carboxylic ester group, and wherein the vinyl copolymer and the cross-linked vinyl polymer each has a molar ratio of the component unit (I) to (II) of from 1 : 20 to 1 : 0.01
- determining the viable cell number of aerobic bacteria in the sample as an output of the oxygen-sensing electrode effected by the contact of the sample with the pyridinium type resin layer.

2. The method for determining viable cell number according to claim 1, wherein the vinyl copolymer and the cross-linked vinyl polymer is in powdery form.

3. The method for determining viable cell number according to anyone of claims 1 or 2, wherein the aqueous sample is contacted with the water-insoluble pyridinium type resin layer for 10 to 30 minutes.

4. An apparatus for determining aerobic bacteria, comprising :
- an oxygen-sensing electrode having an oxygen-sensing portion ;
- a flow cell capable of passing an aqueous sample there-through with the sample being in contact with the oxygen-sensing portion; and
- a water-insoluble pyridinium type resin contained in said flow cell, said water-insoluble pyridinium type resin comprising a vinyl copolymer composed of component units represented by the following formulas (I) and (II), or a cross-linked vinyl polymer formed by cross-linking the polymer composed of the component unit of the formula (I) with a polymeric chain composed of the unit of the formula (II) 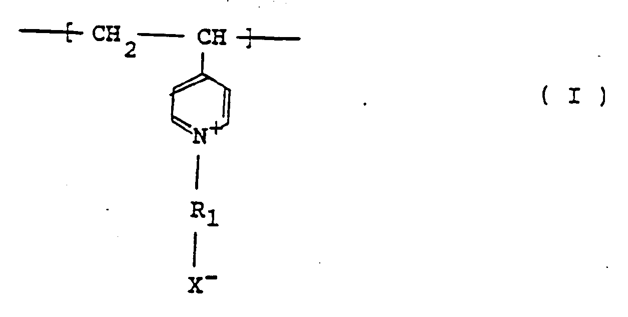 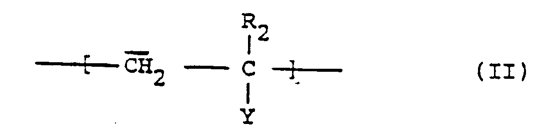 wherein R₁ represents a benzyl group, an alkyl group having C₄ to C₁₆, or an aryl group, R₂ represents an alkyl group having C₁ to C₃, X represents a halogen atom, and Y represent a hydrogen atom, an alkyl group having C₁ to C₃, a benzyl group, an aryl group, an ether group, a carboxyl group, or a carboxylic ester group, and wherein the vinyl copolymer and the cross-linked vinyl polymer each has a molar ratio of the component unit (I) to (II) of from 1 : 20 to 1 : 0.01

5. The apparatus according to claim 4 wherein the vinyl copolymer and the cross-linked vinyl polymer are in powdery form.

## Patentansprüche

1. Verfahren zur Bestimmung der Anzahl lebensfähiger Zellen von aeroben Bakterien, umfassend
- das Aufbringen einer in Wasser unlöslichen Harzschicht vom Pyridinium-Typus auf einen Messabschnitt einer Sauerstoff-Messelektrode;
- das Inberührungbringen einer wässerigen, bakterienhaltigen Probe mit der in Wasser unlöslichen Harzschicht vom Pyridium-Typus, welcher ein Vinylcopolymer, zusammengesetzt aus den Komponenteneinheiten, wie sie durch die folgenden Formeln (I) und (II) dargestellt sind, oder ein vernetztes Vinylpolymer, gebildet durch Vernetzung eines Polymers, zusammengesetzt aus der Komponenteneinheit der Formel (I) mit einer polymeren Kette, zusammengesetzt aus der Einheit der Formel (II) 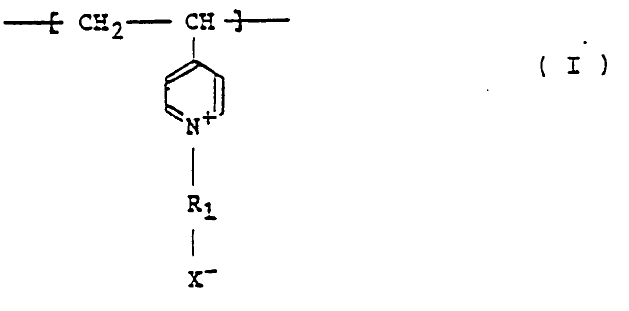 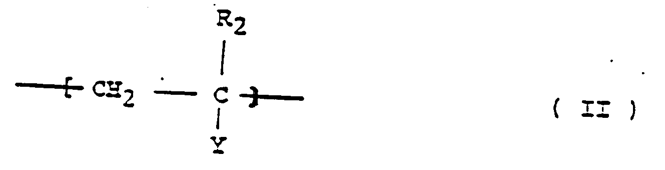 in welcher R₁ eine Benzylgruppe, eine Alkylgruppe mit C₄ bis C₁₆ oder eine Arylgruppe darstellt, R₂ eine Alkylgruppe mit C₁ bis C₃ bedeutet, X ein Halogenatom ist und Y ein Wasserstoffatom, eine Alkylgruppe mit C₁ bis C₃, eine Benzylgruppe, eine Arylgruppe, eine Aethergruppe, eine Carboxylgruppe oder eine Carbonsäureestergruppe darstellt, umfasst, und in welcher das Vinylcopolymer und das vernetzte Vinylpolymer jedes ein molares Verhältnis der Komponenteneinheit (I) zu (II) von 1 : 20 bis 1 : 0,01 aufweist,
- Bestimmung der Anzahl lebensfähiger Zellen der aeroben Bakterien in der Probe als Leistung der Sauerstoff-Messelektrode, bewirkt durch die Berührung der Probe mit der Harzschicht vom Pyridinium-Typus.

2. Verfahren zur Bestimmung der Anzahl lebensfähiger Zellen nach Anspruch 1, in welchen das Vinylcopolymer und das vernetzte Vinylpolymer in Pulverform vorliegen.

3. Verfahren zur Bestimmung der Anzahl lebensfähiger Zellen nach einem der Ansprüche 1 oder 2, in welchem die wässerige Probe mit der in Wasser unlöslichen Harzschicht vom Pyridinium-Typus während 10 bis 30 Minuten in Berührung gebracht wird.

4. Vorrichtung zur Bestimmung aerober Bakterien, umfassend :
- eine Sauerstoff-Messelektrode mit einem Sauerstoff messenden Abschnitt;
- eine Durchflusszelle, welche befähigt ist, eine wässerige Probe durchzulassen, wobei die Probe in Berührung mit dem den Sauerstoff messenden Abschnitt ist, und
- ein in dieser Durchflusszelle enthaltenes, in Wasser unlösliches Harz vom Pyridinium-Typus, wobei das in Wasser unlösliche Harz vom Pyridinium-Typus ein Vinylcopolymer, zusammengesetzt aus Komponenteneinheiten, wie sie durch die folgenden Formeln (I) und (II) dargestellt sind, oder ein vernetztes Vinylpolymer, gebildet durch Vernetzung des Polymers, zusammengesetzt aus der Komponenteneinheit der Formel (I), mit einer polymeren Kette, zusammengesetzt aus der Einheit der Formel II 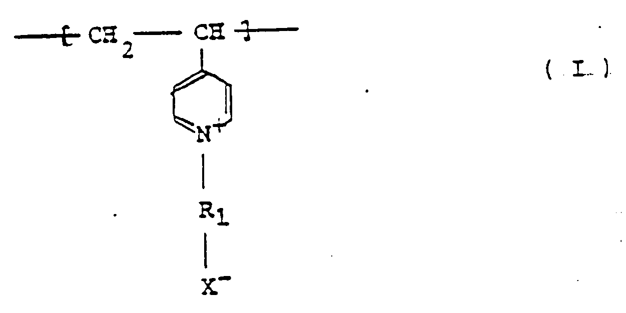 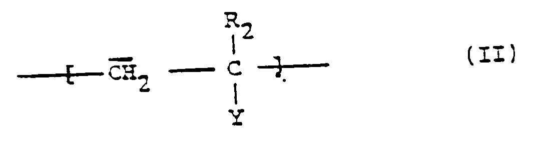 in welcher R₁ eine Benzylgruppe, eine Alkylgruppe mit C₄ bis C₁₆ oder eine Arylgruppe darstellt, R₂ eine Alkylgruppe mit C₁ bis C₃ bedeutet, X ein Halogenatom ist und Y ein Wasserstoffatom, eine Alkylgruppe mit C₁ bis C₃, eine Benzylgruppe, eine Arylgruppe, eine Aethergruppe, eine Carboxylgruppe oder eine Carbonsäureestergruppe darstellt, umfasst, und worin das Vinylcopolymer und das vernetzte Vinylpolymer jedes ein molares Verhältnis der Komponenteneinheit (I) zu (II) von 1 : 20 bis 1 : 0,01 aufweist.

5. Vorrichtung nach Anspruch 4, in welcher das Vinylcopolymer und das vernetzte Vinylpolymeer in Pulverform vorliegen.

## Revendications

1. Procédé pour déterminer le nombre de cellules viables de bactéries aérobies, comprenant :
la mise en place d'une couche de résine de type pyridinium insoluble dans l'eau sur une partie sensible d'une électrode détectant l'oxygène;
la mise en contact d'un échantillon aqueux contenant des bactéries avec la couche de résine de type pyridinium insoluble dans l'eau comprenant un copolymère vinylique composé des unités composantes représentées par les formules suivantes (I) et (II), ou un polymère vinylique réticulé formé par réticulation d'un polymère composé de l'unité composante de formule (I) avec une chaîne polymère composée de l'unité de formule (II) : 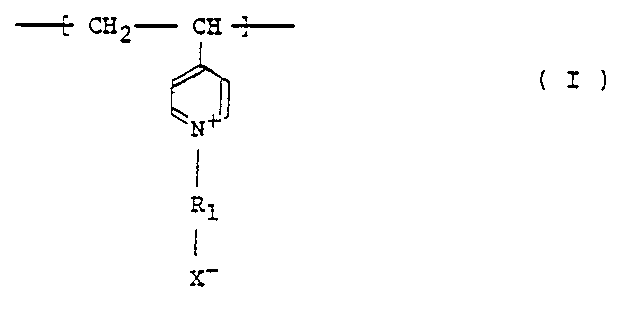 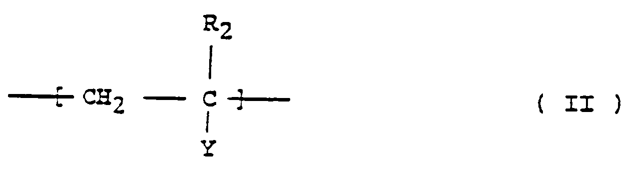 dans lesquelles R₁ représente un groupe benzyle, un groupe alkyle en C₄₋₁₆ ou un groupe aryle, R₂ représente un groupe alkyle en C₁₋₃, X représente un atome d'halogène, et Y représente un atome d'hydrogène, un groupe alkyle en C₁₋₃, un groupe benzyle, un groupe aryle, un groupe éther, un groupe carboxy ou un groupe ester carboxylique, le copolymère vinylique et le polymère vinylique réticulé présentant chacun un rapport molaire des unités composantes (I) à (II) de 1:20 à 1:0,01;
la détermination du nombre de cellules viables de bactéries aérobies dans l'échantillon en tant que sortie de l'électrode détectant l'oxygène déclenchée par contact de l'échantillon avec la couche de résine de type pyridinium.

2. Procédé pour déterminer le nombre de cellules viables suivant la revendication 1, dans lequel le copolymère vinylique et le polymère vinylique réticulé sont sous forme pulvérulente.

3. Procédé pour déterminer le nombre de cellules viables suivant les revendications 1 ou 2, dans lequel l'échantillon aqueux est mis en contact avec la couche de résine de type pyridinium insoluble dans l'eau pendant une période de 10 à 30 minutes.

4. Appareil pour déterminer des bactéries aérobies, comprenant :
une électrode détectant l'oxygène ayant une partie sensible à l'oxygène;
une cellule d'écoulement à travers laquelle un échantillon aqueux peut passer en contact avec la partie sensible l'oxygène; et
une résine de type pyridinium insoluble dans l'eau contenue dans cette cellule d'écoulement, cette résine de type pyridinium insoluble dans l'eau comprenant un copolymère vinylique composé des unités composantes représentées par les formules suivantes (I) et (II), ou un polymère vinylique réticulé formé par réticulation d'un polymère composé de l'unité composante de formule (I) avec une chaîne polymère composée de l'unité de formule (II) : 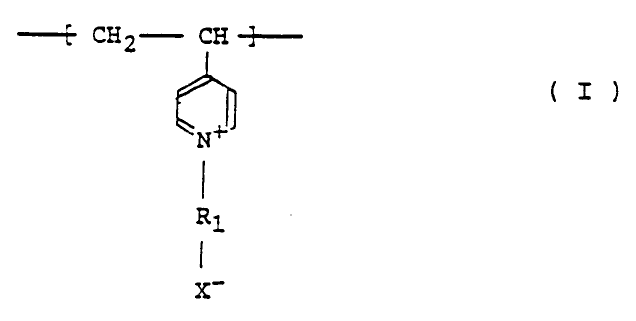 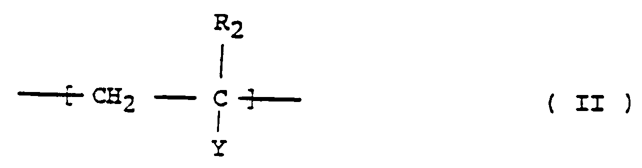 dans lesquelles R₁ représente un groupe benzyle, un groupe alkyle en C₄₋₁₆ ou un groupe aryle, R₂ représente un groupe alkyle en C₁₋₃, X représente un atome d'halogène, et Y représente un atome d'hydrogène, un groupe alkyle en C₁₋₃, un groupe benzyle, un groupe aryle, un groupe éther, un groupe carboxy ou un groupe ester carboxylique, le copolymère vinylique et le polymère vinylique réticulé présentant chacun un rapport molaire des unités composantes (I) à (II) de 1:20 à 1:0,01.

5. Appareil suivant la revendication 4, dans lequel le copolymère vinylique et le polymère vinylique réticulé sont sous forme pulvérulente.
